# EUROPEAN PATENT APPLICATION

(11) **EP 1 051 993 A2**
(43) Date of publication of application: **15.11.2000**
(21) Application number: 00303887.4
(22) Date of filing: 09.05.2000
(51) Int. Cl.: A61P 25/06, A61K 45/06

(54) **5HT1 receptor agonists and either a cox-2 inhibitor or NSAID for the treatment of migraine**

(30) Priority: 14.05.1999 US 134311 P
(71) Applicant: Pfizer Products Inc., Groton, CT 06340-5146 (US)
(72) Inventor: Sands, George Harry, Goton, Connecticut 06340 (US)
(74) Representative: Simpson, Alison Elizabeth Fraser

(57) **Abstract**

The present invention relates to a method of treating migraine in a mammal, including a human, by administering to the mammal a 5HT₁ receptor agonist in combination with either a cyclooxygenase-2 (COX-2) inhibitor or a nonsteroidal antiinflammatory drug (NSAID). It also relates to pharmaceutical compositions containing a pharmaceutically acceptable carrier, a 5HT₁ receptor agonist with either a cyclooxygenase-2 (COX-2) inhibitor or a nonsteroidal antiinflammatory drug (NSAID).

## Description

The present invention relates to a method of treating migraine in a mammal, including a human, by administering to the mammal a 5HT₁ receptor agonist in combination with either a cyclooxygenase-2 (COX-2) inhibitor or a nonsteroidal antiinflammatory drug (NSAID). It also relates to pharmaceutical compositions containing a pharmaceutically acceptable carrier, a 5HT₁ receptor agonist and either a COX-2 inhibitor or NSAID. Examples of agonists of 5HT₁ receptors are agonists of one or more of the 5HT_{1A}, 5HT_{1B}, 5HT_{1C}, 5HT_{1D}, 5HT_{1E}, and 5HT_{1F} receptors.

The combined use of 5HT₁ agonists (e.g. eletriptan, rizatriptan, naratriptan, sumatriptan, zolmitriptan) and either a COX-2 inhibitor or NSAID for the acute treatment of migraine offers enhanced efficacy than currently used therapies.

Symptomatic treatment helps relieve the pain associated with migraine. Abortive treatment targets the pathophysiology of migraine and decreases many of the symptoms of migraine, including pain, nausea, photophobia and phonophobia.

NSAIDS have been shown to help in the symptomatic treatment of migraine headache. Its combination with the abortive treatment of the 5HT₁ agonists is expected to provide an additional effect than the use of either treatment alone.

COX-2 inhibitors have evolved from the NSAIDS and are expected to have similar efficacy with additional safety and tolerability. By selectively inhibiting the COX-2 isoenzyme associated with inflammation and pain, COX-2 inhibitors would be expected to decrease migraine pain with less or no effect on the COX-1 isoenzyme. This isoenzyme maintains gastrointestinal and renal environments. The effect of the NSAIDS on the COX-1 isoenzyme is thought to be responsible for the large incidence of gastrointestinal and renal adverse experiences associated with NSAIDS treatment. Therefore, the use of the COX-2 inhibitors is advantageous with its additional safety and tolerability.

### Summary of the Invention

The present invention relates to pharmaceutical compositions for the treatment of migraine in a mammal, including a human, comprising a 5HT₁ receptor agonist or a pharmaceutically acceptable salt thereof, and either a cyclooxygenase-2 (COX-2) inhibitor or a nonsteroidal antiinflamatory drug (NSAID) and a pharmaceutically acceptable carrier.

This invention also relates to a method of treating migraine in a mammal, including a human, comprising administering to said mammal an amount of a pharmaceutical composition comprising a 5HT₁ receptor agonist or a pharmaceutically acceptable salt thereof, and either a cyclooxygenase-2 (COX-2) inhibitor or a nonsteroidal antiinflamatory drug (NSAID) and a pharmaceutically acceptable carrier, that is effective in treating migraine.

This invention also relates to a method of treating migraine in a mammal, including a human, comprising administering to said mammal a 5HT₁ receptor agonist, or a pharmaceutically acceptable salt thereof, and either a cyclooxygenase-2 (COX-2) inhibitor or a nonsteroidal antiinflamatory drug (NSAID) in amounts that render the combination of such two active agents effective in the treatment or prevention of migraine.

Preferred embodiments of this invention relate to pharmaceutical compositions for the treatment of migraine and methods of treating migraine, as described above, wherein the 5HT₁ receptor agonist is selected from eletriptan, naratriptan, rizatriptan, sumatriptan almotriptan, avitriptan, frovatriptan, alniditan, zolmitriptan, LY 334370, LY 306258, BMS-180048 and BMS-181885.

Other preferred embodiments of this invention relate to pharmaceutical compositions for the treatment of migraine and methods of treating migraine, as described above, wherein the nonsteroidal antiinflamatory drug (NSAID) is selected from diclofenac sodium, nabumetone, naproxen, naproxen sodium, ketorolac and ibuprofen.

Other embodiments of this invention relate to pharmaceutical compositions for the treatment of migraine and methods of treating migraine, as described above, wherein the 5HT₁ receptor agonist is a compound of the formula 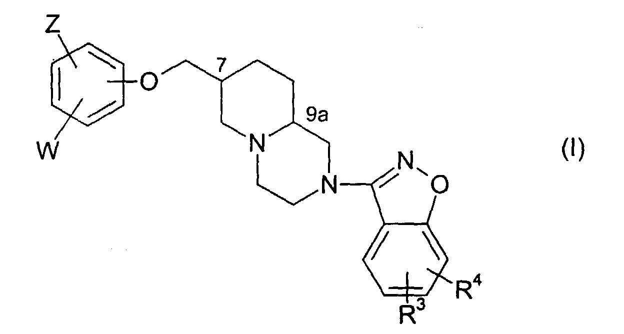 wherein
R³, R⁴, and Z are selected, independently, from hydrogen, halo (e.g., chloro, fluoro, bromo or iodo), (C₁-C₄) alkyl optionally substituted with from one to three fluorine atoms, (C₁-C₄)alkoxy optionally substituted with from one to three fluorine atoms, and (C₁-C₄)alkoxy-(C₁-C₄)alkyl wherein each of the alkyl moieties may optionally be substituted with from one to three fluorine atoms;
W is -CH₂-O-(C₁-C₆) alkyl wherein the alkyl moiety can be straight or branched;
or W is -CH₂NR¹R² wherein R¹ and R² are independently selected from hydrogen and straight or branched (C₁-C₆)alkyl;
or R¹ and R², together with the nitrogen to which they are attached, form a saturated four membered monocyclic ring or a saturated or unsaturated nonaromatic five to seven membered monocyclic ring or a saturated or unsaturated nonaromatic seven to ten membered bicyclic ring which may optionally contain one or two heteroatoms in addition to the nitrogen of NR¹R², wherein said heteroatoms are independently selected from oxygen, nitrogen and sulfur, and wherein from one to three of the ring carbon atoms, or one of the ring nitrogen atoms, may optionally and independently be substituted with straight or branched (C₁-C₄) alkyl, straight or branched (C₁-C₆) alkoxy, straight or branched (C₁-C₃) alkyl-(C₃-C₇) cycloalkyl, hydroxy, amino, cyano, halo, aryl-(straight or branched (C₁-C₃) alkyl) or heteroaryl-(straight or branched (C₁-C₃) alkyl), wherein said aryl is selected from phenyl and naphthyl and said heteroaryl is selected from oxazolyl, isoxazoyl, thiazolyl, isothiazolyl, furanyl, pyrazolyl, pyrrolyl, tetrazolyl, triazolyl, thienyl, imidazolyl, pyrazinyl, pyrazolyl, indolyl, isoindolyl, pyrazinyl, cinnolinyl, pyridinyl and pyrimidinyl;
with the proviso that in any ring formed by NR¹R²: (a) there can be no more than one ring oxygen atom; (b) there can be no hydroxy, alkoxy, alkoxyalkyl, cyano, amino or alkylamino moiety bonded directly to any ring nitrogen atom; and (c) no ring carbon that is double bonded to another ring carbon and not part of an aromatic ring system can be bonded to a ring oxygen atom or ring nitrogen atom;
or a pharmaceutically acceptable salt thereof.

Other embodiments of this invention relate to pharmaceutical compositions for the treatment of migraine and methods of treating migraine, as described above, wherein the cyclooxygenase-2 (COX-2) inhibitor is a compound of the formula 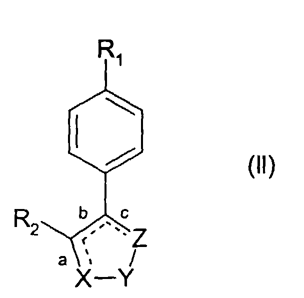 or pharmaceutically acceptable salts thereof wherein:
X-Y-Z- is selected from the group consisting of -C(O)-O-CR⁵(R⁵)- when side b is a double bond, and sides a and c are single bonds; and
R¹ is selected from the group consisting of
   (a) S(O)₂CH₃,
   (b) S(O)₂NH₂,
R² is selected from the group consisting of
   (a) C₁₋₆alkyl,
   (b) C₃, C₄, C₅, C₆ and C₇, cycloalkyl,
   (c) Heteroaryl
   (d) Benzoheteroaryl
   (e) Mono- or di-substituted phenyl wherein the substituent is selected from the group consisting of
      (1) hydrogen,
      (2) halo,
      (3) C₁₋₆alkoxy,
      (4) C₁₋₆alkylthio,
      (5) CN,
      (6) CF₃,
      (7) C₁₋₆alkyl,
      (8) N₃,
      (9) -CO₂H,
      (10)-CO₂-C₁₋₄alkyl,
      (11)-C(R⁵)(R⁶)-OH,
      (12)-C(R⁵)(R⁶)-O-C₁₋₄alkyl, and
      (13)-C₁₋₆alkyl-CO₂R⁵;
R⁵, R⁵ and R⁶ are each independently selected from the group consisting of
   (a) hydrogen,
   (b) C₁₋₆alkyl,
or R⁵ and R⁶ together with the carbon to which they are attached from a saturated monocyclic carbon ring is 3, 4, 5, 8 or 7 atoms.

### Detailed Description of the Invention

The following patents and patent applications exemplify 5HT₁ agonists that can be used, in combination with either a cyclooxygenase-2 (COX-2) inhibitor or a nonsteroidal antiinflamatory drug (NSAID), in the pharmaceutical compositions and methods of this invention, and refer to methods of preparing the same: U.S. Patent 5,545,644, issued August 13, 1996; European Patent 776,323, granted February 11, 1998; United Sates Patent 5,618,834, issued April 8, 1997; World Patent Application PCT/EP98/04176, which designates the United States and was filed on July 1, 1998; European Patent 503,440, granted June 18, 1998; United States Patent 4,816,470, issued March 28, 1989; Japanese Patent 9,423,197, granted March 30, 1994; Canadian Patent 1,241,004, granted August 23, 1988; European Patent 497,512, granted Sepember 24, 1997; United States Patent 5,300,506, issued April 15, 1994; European Patent Application 711,769, published May 15, 1996; World Patent Application WO 94/2460, published February 3, 1994; United States Patent 5,541,180, issued July 30, 1996; European Patent Application 591,280, published April 13, 1994; European Patent 639,192, granted May 15, 1996; European Patent Application 674,621, published October 4, 1995 and European Patent 486,666, granted August 13, 1997. The foregoing patents and patent applications are incorporated herein by reference in their entireties.

The following references relate to the pharmacological properties of certain of the 5HT₁ agonists mentioned above as being employed in preferred embodiments of this invention: Robert et al., Cephalagia 18(6): 406, July/August 1998; Marathe et al., Biopharm. Drug Dispos. 19(6): 381-94, September 1998; Saxena et al., Eur. J. Pharmacol. 351(3): 329-39, 26 June 1998; Goldstein et al., Cephalagia 18(6): 410, July/August 1998; Buchan et al., Cephalagia 18(6): 410, July/August 1998; Block et al., Cephalagia 18(6): 409-10, July/August 1998; and Sheftell et al., Cephalagia 18(6): 403-4, July/August 1998; Perry et al., Drugs (New Zealand) 55(6):889-922, June 1998; Bomhof et al., Cephalagia (Norway) 18(1): 33-7, January 1998; Klasson et al., Headaches (United States) 37(10): 640-5, Nov./Dec. 1997; Goldstein et al., Cephalagia (Norway) 16(7): 497-502, November 1996; Parsons et al., J. Cardiovasc. Pharmacol. (United States) 32(2): 220-4, August 1998; and Schoenen J., Curr. Opin. Neurol. 10(3): 237-43, June 1997. These references are incorporated herein by reference in their entireties.

The following patents and patent applications exemplify cyclooxygenase-2 (COX-2) inhibitors that can be used, in combination with a 5HT₁ agonists, in the pharmaceutical compositions and methods of this invention, and refer to methods of preparing the same: United States Patent 5,817,700, issued October 6, 1998; World Patent Application WO97/28121, published August 7, 1997; United States Patent 5,767,291, issued June 16, 1998; United States Patent 5,436,265, issued July 25 1995; United States Patent 5,474,995, issued December 12, 1995; United States Patent 5,536,752, issued July 16, 1996; United States Patent 5,550,142, issued August 27, 1996; United States Patent 5,604,260, issued February 18, 1997; United States Patent 5,698,584, issued December 16, 1997; United States Patent 5,710,140, issued January 20, 1998; United States Patent 5,840,746, issued November 24, 1998; Great Britain Patent Application 986430, filed March 25, 1998; World Patent Application WO97/28120, published August 7, 1997; Great Britain Patent Application 9800689, filed January 14, 1998; Great Britain Patent Application 9800688, filed January 14, 1998; World Patent Application WO94/14977, published July 7, 1994; World Patent Application WO98/43966, published October 8, 1998; World Patent Application WO98/03484, published January 29, 1998; World Patent Application WO98/41516, published September 24, 1998; World Patent Application WO98/41511, published September 24, 1998; Great Britain Patent Application 2,319,032, issued May 13, 1998; World Patent Application WO96/37467, published November 28, 1996; World Patent Application WO96/37469, published November 28, 1996; World Patent Application WO96/36623, published November 21, 1996; World Patent Application WO98/00416, published January 8, 1998; World Patent Application WO97/44027, published November 27, 1997; World Patent Application WO97/44028, published November 27, 1997; World Patent Application WO96/23786, published August 8, 1996; World Patent Application WO97/40012, published October 30, 1997; World Patent Application WO96/19469, published June 27, 1996; World Patent Application WO97/36863, published October 9, 1997; World Patent Application WO97/14691, published April 24, 1997; World Patent Application WO97/11701, published April 3, 1997; World Patent Application WO96/13483, published May 9, 1996; World Patent Application WO96/37468, published November 28, 1996; World Patent Application WO96/06840, published March 7, 1996; World Patent Application WO94/26731, published November 24, 1994; World Patent Application WO94/20480, published September 15, 1994; United States Patent 5,006,549, issued April 9, 1991; United States Patent 4,800,211, issued January 24, 1989; United States Patent 4,782,080, issued November 1, 1988; United States Patent 4,720,503, issued January 19, 1988; United States Patent 4,760,086, issued July 26, 1988; United States Patent 5,068,248, issued November 26, 1991; United States Patent 5,859,257, issued January 12, 1999; World Patent Application WO98/47509, published October 29, 1998; World Patent Application WO98/47890, published October 29, 1998; World Patent Application WO98/43648, published October 8, 1998; World Patent Application WO98/25896, published June 18, 1998; World Patent Application WO98/22101, published May 28, 1998; World Patent Application WO98/16227, published April 23, 1998; World Patent Application WO98/06708, published February 19, 1998; World Patent Application WO97/38986, published October 23, 1997; United States Patent 5,663,180, issued September 2, 1997; World Patent Application WO97/29776, published August 21, 1997; World Patent Application WO97/29775, published August 21, 1997; World Patent Application WO97/29774, published August 21, 1997; World Patent Application WO97/27181, published July 31, 1997; World Patent Application WO95/11883, published May 4, 1995; World Patent Application WO97/14679, published April 24, 1997; World Patent Application WO97/11704, published April 3, 1997; World Patent Application WO96/41645, published December 27, 1996; World Patent Application WO96/41626, published December 27, 1996; World Patent Application WO96/41625, published December 27, 1996; World Patent Application WO96/38442, published December 5, 1996; World Patent Application WO96/38418, published December 5, 1996; World Patent Application WO96/36617, published November 21, 1996; World Patent Application WO96/24585, published August 15, 1996; World Patent Application WO96/24584, published August 15, 1996; World Patent Application WO96/16934, published June 6, 1996; World Patent Application WO96/03385, published February 8, 1996; World Patent Application WO96/12703, published May 2, 1996; World Patent Application WO96/09304, published March 28, 1996; World Patent Application WO96/09293, published March 28, 1996; World Patent Application WO96/03392, published February 8, 1996; World Patent Application WO96/03388, published February 8, 1996; World Patent Application WO96/03387, published February 8, 1996; World Patent Application WO96/02515, published February 1, 1996; World Patent Application WO96/02486, published February 1, 1996; United States Patent 5,476,944, issued December 19, 1995; World Patent Application WO95/30652, published November 16, 1995; United States Patent 5,451,604, published September 19, 1995; World Patent Application WO95/21817, published August 17, 1995; World Patent Application WO95/21197, published August 10, 1995; World Patent Application WO95/15315, published June 8, 1995; United States Patent 5,504, 215, issued April 2, 1996; United States Patent 5,508,426, issued April 16, 1996; United States Patent 5,516,907, issued May 14, 1996; United States Patent 5,521,207, issued May 28, 1998; United States Patent 5,753,688, issued May 19, 1998; United States Patent 5,760,068, issued June 2, 1998; United States Patent 5,420,343, issued May 30, 1995; World Patent Application WO95/30656, published November 16, 1995; United States Patent 5,393,790, issued February 28, 1995; and World Patent Application WO94/27980, published February 8, 1994. The foregoing patents and patent applications are incorporated herein by reference in their entireties.

The term "treating", as used herein, refers to retarding or reversing the progress of or alleviating or preventing either the disorder or condition to which the term "treating" applies, or one or more symptoms of such disorder or condition. The term "treatment", as used herein, refers to the act of treating a disorder or condition, as the term "treating" is defined above.

This invention relates both to methods of treating migraine in which the 5HT₁ receptor agonist, with either a cyclooxygenase-2 (COX-2) inhibitor or a nonsteroidal antiinflamatory drug (NSAID), are administered together, as part of the same pharmaceutical composition, as well as to methods in which these two active agents are administered separately, as part of an appropriate dose regimen designed to obtain the benefits of the combination therapy. The appropriate dose regimen, the amount of each dose administered, and the intervals between doses of the active agents will depend upon the 5HT₁ agonist, COX-2 inhibitor or NSAID, being used, the type of pharmaceutical formulations being used, the characteristics of the subject being treated and the severity of the migraine. Generally, in carrying out the methods of this invention, the 5HT₁ receptor agonist will be administered orally to an average 70 kg adult human in an amount ranging from about 0.5 to about 100 mg per day, in single or divided doses, and the COX-2 inhibitor or NSAID will be administered in single or divided doses. COX-2 inhibitors will generally be administered in amounts ranging from about 10 to about 300 mg per day, depending on the COX-2 inhibitor, severity of the headache and the route of administration. NSAIDS will generally be administered in amounts ranging from about 15 to about 2,000 mg per day, depending on the NSAID, severity of the headache and the route of administration. The COX-2 inhibitors and NSAIDS can be administered orally, intranasally, intravenously, as a rectal suppository or using a "flash" formulation (i.e., allowing the medication to dissolve in the mouth without the need to use water.)

The following tables exemplify preferred dosage ranges of certain specific 5HT₁ agonists when used in combination with either cyclooxygenase-2 (COX-2) inhibitors or nonsteroidal antiinflammatory drugs (NSAIDS).

**TABLE 1**

| **5HT**_{**1**} **AGONIST** | **DOSAGE RANGE FOR MEDICATION TAKEN** | **DOSAGE RANGE FOR MEDICATION TAKEN INTRANASALLY (mg)** |
|---|---|---|
| Eletriptan | 20 to 80 | - |
| Rizatriptan | 5 to 10 | - |
| Zolmitriptan | 1 to 5 | - |
| Sumatriptan | 25 to 100 | 5 to 20 |
| Naratriptan | 1 to 5 | - |
| Dihydroergotamine | - | 0.5 to 2 |
| Ergotamine | 0.5 to 2 | - |

**TABLE 2**

| **COX2-Inhibitors** | **DOSAGE RANGE (mg) P.O.** |
|---|---|
| VIOXX™ | 10 to 100 |

**TABLE 3**

| **NSAIDS** | **DOSAGE RANGE (mg)** |
|---|---|
| Diclofenac sodium | 50 to 100 |
| Nabumetone | 500 to 2000 |
| Naproxen | 200 to 825 |
| Naproxen sodium | 200 to 825 |
| Ketorolac | 15 to 60 I.M. or I.V.; 10 to 30 P.O. |
| Ibuprofen | 200 to 800 |

The 5HT₁ receptor agonists with either a cyclooxygenase-2 (COX-2) inhibitor or a nonsteroidal antiinflammatory drug (NSAID), that are employed in the pharmaceutical compositions and methods of this invention, and their pharmaceutically acceptable salts, may be administered alone (two active agents administered together or separately) or in combination with pharmaceutically acceptable carriers or diluents. They may be formulated in a conventional manner using one or more pharmaceutically acceptable carriers. Such compounds may be adminstered orally, buccally, intranasally, parenterally (e.g., intravenously, intramuscularly or subcutaneously) or rectally, or in a form suitable for administration by inhalation or insufflation.

For oral administration (two active agents administered together or separately), the pharmaceutical compositions may take the form of for example, tablets or capsules prepared by conventional means with pharmaceutically acceptable excipients such as binding agents e.g., pregelatinised maize starch, polyvinylpyrrolidone or hydroxypropyl methylcellulose); fillers (e.g., lactose, microcrystalline cellulose or calcium phosphate), lubricants (e.g., magnesium stearate, talc or silica); disintegrants (e.g., potato starch or sodium starch glycollate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration the composition (two active agents administered together or separately) may take the form of tablets or lozenges formulated in a conventional manner.

The 5HT₁ agonists of the invention and their salts with either a cyclooxygenase-2 (COX-2) inhibitor or a nonsteroidal antiinflammatory drug (NSAID), may be formulated for parenteral administration (two active agents administered together or separately) by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulating agents such as suspending, stabilizing and/or dispersing agents.

Alternatively, the active ingredients (two active agents administered together or separately) may be in powder form for reconstitution with a suitable vehicle, e.g., sterile pyrogen-free water, prior to use.

The 5HT₁ agonists of this invention and their salts with either a cyclooxygenase-2 (COX-2) inhibitor or a nonsteroidal antiinflammatory drug (NSAID), may also be formulated (two active agents administered together or separately) in rectal compositions such as suppositories or retention enemas, e.g., containing conventional suppository bases such as cocoa butter or other glycerides.

For intranasal administration or administration by inhalation, the active compounds of the invention (two active agents administered together or separately) are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol , the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

Aerosol formulations (two active agents administered together or separately) for the treatment of migraine in the average adult human are preferably made so that each metered dose or "puff' of aerosol contains 20 µg to 1000 µg of the active compounds of the invention. The overall daily dose with an aerosol will generally be within the range of about 100 µg to 10 mg. Administration may be several times daily, for example, 2, 3, 4 or 8 times, giving, for example, 1, 2 or 3 doses each time.

The 5-HT₁ receptor agonist activity of a compound or salt can be measured in in vitro receptor binding assays as described for the 5-HT_{1A} receptor, using rat cortex as the receptor source and [³H]8-OH-DPAT as the radioligand (D. Hoyer et al., Europ. J. Pharmacol., 1985; 118: 13), and as described for the 5-HT_{1D} receptor, using bovine caudate as the receptor source and [³H]5-HT as the radioligand (R.E. Heuring and S. J. Peroutka, J. Neuroscience, 1987; 7: 894).

The in vitro activity of a compound at the 5-HT_{1D} binding site may be determined according to the following procedure. Bovine caudate tissue is homogenized and suspended in 20 volumes of a buffer containing 50 mM TRIS·hydrochloride (tris[hydroxymethyl]aminomethane hydrochloride) at a pH of 7.7. The homogenate is then centrifuged at 45,000G for 10 minutes. The supernatant is then discarded and the resulting pellet resuspended in approximately 20 volumes of 50 mM TRlS·hydrochloride buffer at pH 7.7. This suspension is then pre-incubated for 15 minutes at 37°C, after which the suspension is centrifuged again at 45,000G for 10 minutes and the supernatant discarded. The resulting pellet (approximately 1 gram) is resuspended in 150 ml of a buffer of 15 mM TRlS·hydrochloride containing 0.01 percent ascorbic acid with a final pH of 7.7 and also containing 10 mM pargyline and 4 mM calcium chloride (CaCl₂). The suspension is kept on ice at least 30 minutes prior to use.

The inhibitor, control or vehicle is then incubated according to the following procedure. To 50 ml of a 20 percent dimethylsulfoxide (DMSO)/80 percent distilled water solution is added 200 ml of tritiated 5-hydroxytryptamine (2 nM) in a buffer of 50 mM TRIS·hydrochloride containing 0.01 percent ascorbic acid at pH 7.7 and also containing 10 mM pargyline and 4 mM calcium chloride, plus 100 nM of 8-hydroxy-DPAT (dipropylaminotetraline) and 100 nM of mesulergine. To this mixture is added 750 ml of bovine caudate tissue, and the resulting suspension is vortexed to ensure a homogenous suspension. The suspension is then incubated in a shaking water bath for 30 minutes at 25°C. After incubation is complete, the suspension is filtered using glass fiber filters (e.g., Whatman GF/B-filters). The pellet is then washed three times with 4 ml of a buffer of 50 mM TRlS·hydrochloride at pH 7.7. The pellet is then placed in a scintillation vial with 5 ml of scintillation fluid (aquasol 2) and allowed to sit overnight. The percent inhibition can be calculated for each dose of the compound. An IC₅₀ value can then be calculated from the percent inhibition values.

The ability of a compound or salt to bind to 5-HT_{1A} receptors can be determined according to the following procedure. Rat brain cortex tissue is homogenized and divided into samples of 1 gram lots and diluted with 10 volumes of 0.32 M sucrose solution. The suspension is then centrifuged at 900G for 10 minutes and the supernatant separated and recentrifuged at 70,000G for 15 minutes. The supernate is discarded and the pellet resuspended in 10 volumes of 15 mM TRIS·hydrochloride at pH 7.5. The suspension is allowed to incubate for 15 minutes at 37°C. After pre-incubation is complete, the suspension is centrifuged at 70,000G for 15 minutes and the supernate discarded. The resulting tissue pellet is resuspended in a buffer of 50 mM TRIS·hydrochloride at pH 7.7 containing 4 mM of calcium chloride and 0.01 percent ascorbic acid. The tissue is stored at -70°C until ready for an experiment. The tissue can be thawed immediately prior to use, diluted with 10 mm pargyline and kept on ice.

The tissue is then incubated according to the following procedure. Fifty microliters of control, inhibitor, or vehicle (1 percent DMSO final concentration) is prepared at various dosages. To this solution is added 200ml of tritiated DPAT at a concentration of 1.5 nM in a buffer of 50 mM TRIS·hydrochloride at pH 7.7 containing 4 mM calcium chloride, 0.01 percent ascorbic acid and pargyline. To this solution is then added 750 ml of tissue and the resulting suspension is vortexed to ensure homogeneity. The suspension is then incubated in a shaking water bath for 30 minutes at 37°C. The solution is then filtered, washed twice with 4 ml of 10 mM TRIS·hydrochloride at pH 7.5 containing 154 mM of sodium chloride. The percent inhibition is calculated for each dose of the compound, control or vehicle. IC₅₀ values are calculated from the percent inhibition values.

The agonist and antagonist activities compounds at 5-HT_{1A} and 5-HT_{1D} receptors can be determined using a single saturating concentration according to the following procedure. Male Hartley guinea pigs are decapitated and 5-HT_{1A} receptors are dissected out of the hippocampus, while 5-HT_{1D} receptors are obtained by slicing at 350 mM on a McIlwain tissue chopper and dissecting out the substantia nigra from the appropriate slices. The individual tissues are homogenized in 5 mM HEPES buffer containing 1 mM EGTA (pH 7.5) using a hand-held glass-Teflon® homogenizer and centrifuged at 35,000 x g for 10 minutes at 4°C. The pellets are resuspended in 100 mM HEPES buffer containing 1 mM EGTA (pH 7.5) to a final protein concentration of 20 mg (hippocampus) or 5 mg (substantia nigra) of protein per tube. The following agents are added so that the reaction mix in each tube contained 2.0 mM MgCl₂, 0.5 mM ATP, 1.0 mM cAMP, 0.5 mM IBMX, 10 mM phosphocreatine, 0.31 mg/ml creatine phosphokinase, 100 mM GTP and 0.5-1 microcuries of [32P]-ATP (30 Ci/mmol: NEG-003 - New England Nuclear). Incubation is initiated by the addition of tissue to siliconized microfuge tubes (in triplicate) at 30°C for 15 minutes. Each tube receives 20 mL tissue, 10 mL drug or buffer (at 10X final concentration), 10mL 32 nM agonist or buffer (at 10X final concentration), 20mL forskolin (3 mM final concentration) and 40 mL of the preceding reaction mix. Incubation is terminated by the addition of 100 mL 2% SDS, 1.3 mM cAMP, 45 mM ATP solution containing 40,000 dpm [³H]-cAMP (30 Ci/mmol: NET-275 - New England Nuclear) to monitor the recovery of cAMP from the columns. The separation of [³²P]-ATP and [³²P]-cAMP is accomplished using the method of Salomon et al., Analytical Biochemistry, 1974, 58, 541-548. Radioactivity is quantified by liquid scintillation counting. Maximal inhibition is defined by 10 mM (R)-8-OH-DPAT for 5-HT_{1A} receptors, and 320 nM 5-HT for 5-HT_{1D} receptors. Percent inhibitions by the test compounds are then calculated in relation to the inhibitory effect of (R)-8-OH-DPAT for 5-HT_{1A} receptors or 5-HT for 5-HT_{1D} receptors. The reversal of agonist induced inhibition of forskolin-stimulated adenylate cyclase activity is calculated in relation to the 32 nM agonist effect.

Compounds can be tested for in vivo activity for antagonism of 5-HT_{1D} agonist-induced hypothermia in guinea pigs according to the following procedure.

Male Hartley guinea pigs from Charles River, weighing 250-275 grams on arrival and 300-600 grams at testing, serve as subjects in the experiment. The guinea pigs are housed under standard laboratory conditions on a 7 a.m. to 7 p.m. lighting schedule for at least seven days prior to experimentation. Food and water are available ad libitum until the time of testing.

The compounds of the invention can be administered as solutions in a volume of 1 ml/kg. The vehicle used is varied depending on compound solubility. Test compounds are typically administered either sixty minutes orally (p.o.) or 0 minutes subcutaneously (s.c.) prior to a 5-HT_{1D} agonist, such as [3-(1-methylpyrrolidin-2-ylmethyl)-1H-indol-5-yl]-(3-nitropyridin-3-yl)-amine, which can be prepared as described in PCT publication WO93/111 06, published June 10, 1993 which is administered at a dose of 5.6 mg/kg, s.c. Before a first temperature reading is taken, each guinea pig is placed in a clear plastic shoe box containing wood chips and a metal grid floor and allowed to acclimate to the surroundings for 30 minutes. Animals are then returned to the same shoe box after each temperature reading. Prior to each temperature measurement each animal is firmly held with one hand for a 30-second period. A digital thermometer with a small animal probe is used for temperature measurements. The probe is made of semi-flexible nylon with an epoxy tip. The temperature probe is inserted 6 cm. into the rectum and held there for 30 seconds or until a stable recording is obtained. Temperatures are then recorded.

In p.o. screening experiments, a "pre-drug" baseline temperature reading is made at -90 minutes, the test compound is given at -60 minutes and an additional -30 minute reading is taken. The 5-HT_{1D} agonist is then administered at 0 minutes and temperatures are taken 30, 60, 120 and 240 minutes later.

In subcutaneous screening experiments, a pre-drug baseline temperature reading is made at -30 minutes. The test compound and 5-HT_{1D} agonists are given concurrently and temperatures are taken at 30, 60, 120 and 240 minutes later.

Data are analyzed with two-way analysis of variants with repeated measures in Newman-Keuls post hoc analysis.

The 5-HT₁ agonist activity can be determined by the in vitro receptor binding assays, as described for the 5-HT_{1A} receptor using rat cortex as the receptor source and [³H]-8-OH-DPAT as the radioligand [D. Hoyer et al. Eur. J. Pharm., 118, 13 (1985)] and as described for the 5-HT_{1D} receptor using bovine caudate as the receptor source and [3H]serotonin as the radioligand [R.E. Heuring and S.J. Peroutka, J. Neuroscience, 7, 894 (1987)]. Of the active compounds tested, all exhibited an IC₅₀ in either assay of 1 mM or less.

Compounds and salts can be evaluated as anti-migraine agents by testing the extent to which they mimic sumatriptan in contracting the dog isolated saphenous vein strip (P.P.A. Humphrey et al., Br. J. Pharmacol., 1988; 94: 1128.). This effect can be blocked by methiothepin, a known serotonin antagonist. Sumatriptan is known to be useful in the treatment of migraine and produces a selective increase in carotid vascular resistance in the anaesthetized dog. It has been suggested that this is the basis of its efficacy by Fenwick et al., British Journal of Pharmacology., 1989; 96: 83.

The activity of the COX-2 inhibitors of the present invention may be demonstrated by the following assays.

### Human cell based COX-1 assay

Human peripheral blood is obtained from healthy volunteers and diluted to 1/10 volume with 3.8% sodium citrate solution. The platelet-rich plasma is immediately obtained and washed with 0.14 M sodium chloride containing 12 mM Tris-HCl (pH 7.4) and 1.2 mM EDTA. Platelets are then washed with platelet buffer (Hanks buffer (Ca free) containing 0.2% BSA and 20 mM Hepes). Finally, the human washed platelets (HWP) are suspended in platelet buffer at the concentration of 2.85 x 10⁸ cells/ml is stored at room temperature until use. The HWP suspension (70 µl aliquots, final 2.0 x 10⁷ cells/ml) is placed in a 96-well U bottom plate and 10 µl aliquots of 12.6 mM CaCl2 added. Platelets are incubated with A23187 (final 10 µM, Sigma) with test compound (0.1 - 100 µM) dissolved in DMSO (final concentration; less than 0.01%) at 37 °C for 15 minutes. The reaction is stopped by addition of EDTA (final 7.7 mM) and TxB2 in the supernatant quantitated by using a radioimmunoassay kit (Amersham) according to the manufacturer's procedure. *Human cell based* COX-2 assay

### Inhibition of COX-2 activity after induction of COX-2 by hlL-1β

The human cell based COX-2 assay is carried out as previously described (Moore *et al., Inflam. Res*., **45**, 54, 1996). Confluent human umbilical vein endothelial cells (HUVECs, Morinaga) in a 96-well U bottom plate are washed with 100 µl of RPMl1640 containing 2% FCS and incubated with hlL-1β (final concentration 300 U/ml, R & D Systems) at 37 °C for 24 hr. After washing, the activated HUVECs are stimulated with A23187 (final concentration 30 µM) in Hanks buffer containing 0.2% BSA, 20 mM Hepes and test compound (0.1 nM - 100 µM) dissolved in DMSO (final concentration; less than 0.01%) at 37 °C for 15 minutes. 6-Keto-PGF1α, stable metabolite of PGl2, in the supernatant is quantitated after adequate dilution by using a radioimmunoassay kit (Amersham) according to the manufacturer's procedure.

### Inhibition of COX-2 during the induction phase

Confluent human umbilical vein endothelial cells (HUVECs, Morinaga) in a 96-well U bottom plate are washed with 100 µl of RPMl1640 containing 2% FCS and test compound (0.1 nM - 100 µM) dissolved in DMSO (final concentration; less than 0.01%), and incubated with hlL-1β (final concentration 300 U/ml, R & D Systems) at 37 °C for 24 hr. After washing, the HUVECs are stimulated with A23187 (final concentration 30 µM) in Hanks buffer containing 0.2% BSA and 20 mM Hepes at 37 °C for 15 minutes. 6-Keto-PGF1α, a stable metabolite of PGl2, in the supernatant is quantitated after adequate dilution by using a radioimmunoassay kit (Amersham) according to the manufacturers procedure.

### In vivo assays

### Carrageenan induced foot edema in rats

Male Sprague-Dawley rats (5 weeks old, Charles River Japan) are fasted overnight. A line is drawn using a marker above the ankle on the right hind paw and the paw volume (V0) was measured by water displacement using a plethysmometer (Muromachi). Animals are given orally either vehicle (0.1 % methyl cellulose or 5% Tween 80) or a test compound (2.5 ml per 100 grams body weight). One hour later, the animals are then injected intradermally with λ-carrageenan (0.1 ml of 1% w/v suspension in saline, Zushikagaku) into right hind paw (*Winter et al., Proc. Soc. Exp. Biol. Med*., **111**, 544, 1962; Lombardino *et al*, *Arzneim. Forsch*., **25**, 1629, 1975) and three hours later, the paw volume (V3) is measured and the increase in volume (V3-V0) calculated. Since maximum inhibition attainable with classical NSAIDs is 60-70%, ED30 values are calculated.

### Gastric ulceration in rats

The gastric ulcerogenicity of test compound is assessed by a modification of the conventional method (Ezer *et al, J. Pharm. Pharmacol*, **28**, 655, 1976; Cashin *et al*., J. *Pharm. Pharmacol*, **29**, 330 - 336, 1977). Male Sprague-Dawley rats (5 weeks old, Charles River Japan), fasted overnight, are given orally either vehicle (0.1% methyl cellulose or 5% Tween 80) or a test compound (1 ml per 100 grams body weight). Six hours after, the animals are sacrificed by cervical dislocation. The stomachs are removed and inflated with 1% formalin solution (10 ml). Stomachs are opened by cutting along the greater curvature. From the number of rats that showed at least one gastric ulcer or haemorrhaging erosion (including ecchymosis), the incidence of ulceration is calculated. Animals do not have access to either food or water during the experiment.

### Data Analysis

Statistical program packages, SYSTAT (SYSTAT, INC.) and StatView (Abacus Cencepts, Inc.) for Macintosh is used. Differences between test compound treated group and control group are tested for using ANOVA. The IC50 (ED30) values are calculated from the equation for the log-linear regression line of concentration (dose) versus percent inhibition.

COX-2 selectivity can be determined by ratio in terms of IC₅₀, value of COX-1 inhibition to COX-2 inhibition. In general, it can be said that a compound showing a COX-1/COX-2 inhibition ratio of more than 2 has good COX-2 selectivity.

## Claims

1. A pharmaceutical composition for the treatment of migraine comprising a 5HT₁ receptor agonist, with either a cyclooxygenase-2 inhibitor or a nonsteroidal antiinflammatory drug (NSAID), and a pharmaceutically acceptable carrier.

2. A pharmaceutical composition according to claim 1, wherein the 5HT₁ receptor agonist is selected from eletriptan, rizatriptan, zolmitriptan, sumatriptan and naratriptan.

3. A pharmaceutical composition according to claim 1, wherein the nonsteroidal antiinflammatory drug is selected from diclofenal sodium, nabumetone, naproxen sodium, ketorolac and ibuprofen.

4. A method of treating migraine in a mammal, comprising administering to said mammal an antimigraine effective amount of a pharmaceutical composition according to claim 1.

5. A method of treating migraine in a mammal, comprising administering to said mammal a 5HT₁ receptor agonist with either a cyclooxygenase-2 inhibitor or a nonsteroidal antiinflammatory drug, in amounts that render the combination of such two active agents effective in the treatment of migraine.

6. A method according to claim 5, wherein the 5HT₁ receptor agonist is selected from eletriptan, rizatriptan, zolmitriptan sumatriptan and naratriptan.

7. A method according to claim 5, wherein the nonsteroidal antiinflammatory drug is selected from diclofenal sodium, nabumetone, naproxen sodium, ketorolac and ibuprofen.

8. A method according to claim 5, wherein the 5HT₁ receptor agonist and the cyclooxygenase-2 inhibitor are administered separately according to a dose regimen that renders the combination of the separately administered active agents effective in the treatment of migraine.

9. A method according to claim 5, wherein the 5HT₁ receptor agonist and the cyclooxygenase-2 inhibitor are administered together according to a dose regimen that renders the combination of the administered active agents effective in the treatment of migraine.

10. A method according to claim 5, wherein the 5HT₁ receptor agonist and the nonsteroidal antiinflammatory drug are administered separately according to a dose regimen that renders the combination of the separately administered active agents effective in the treatment of migraine.

11. A method according to claim 5, wherein the 5HT₁ receptor agonist and the nonsteroidal antiinflammatory drug are administered together according to a dose regimen that renders the combination of the administered active agents effective in the treatment of migraine.

12. A method according to claim 5, wherein the 5HT₁ receptor agonist is administered in an amount from about .05 mg to about 100 mg per day and the cyclooxygenase-2 inhibitor is administered in an amount from about 10 mg to about 300 mg per day.

13. A method according to claim 5, wherein the 5HT₁ receptor agonist is administered in an amount from about 0.5 mg to about 100 mg per day and the nonsteriodal antiinflammatory drug is administered in an amount from about 15 mg to about 2,000 mg per day.
